# EUROPEAN PATENT APPLICATION

(11) **EP 4 649 964 A1**
(43) Date of publication of application: **19.11.2025**
(21) Application number: 24213380.9
(22) Date of filing: 15.11.2024
(51) Int. Cl.: A61K 47/69, A61P 37/04, C12N 15/88

(54) **COMPOSITE LIPID NANOPARTICLE AND PREPARATION METHOD THEREOF, RNA VACCINE, DRUG AND USE**

(30) Priority: 13.05.2024 CN 202410590037
(71) Applicant: Bisheng (Beijing) Biotechnology Co., Ltd., Beijing (CN)
(72) Inventor: Cao, Yuhong, Beijing (CN); Xu, Hu, Beijing (CN); Li, Tianyao, Beijing (CN)
(74) Representative: Murgitroyd & Company

(57) **Abstract**

The present disclosure belongs to the technical field of lipid nanoparticle biological application, and discloses a composite lipid nanoparticle and a preparation method thereof, an ribose nucleic acid (RNA) vaccine, a drug and use. The composite lipid nanoparticle provided by the present disclosure includes a lipid composition and a recombinant antibody protein containing a streptavidin tag; and the lipid composition comprises an ionizable lipid, an auxiliary lipid, cholesterol, a polyethylene glycol lipid and a biotinylated polyethylene glycol lipid in a mass ratio of (40-90):(0.1-20):(20-50):(0.5-10):(0.5-10). Through strong interaction between the recombinant antibody protein with the streptavidin tag and the biotinylated polyethylene glycol lipid in the composite lipid nanoparticle, the composite lipid nanoparticle achieves safe, convenient, rapid, efficient, and stable linkage between a lipid nanoparticle (LNP) and a recombinant antibody protein, and the recombinant antibody protein can be adaptively replaced according to target cells and/or tissues, with high flexibility and applicability. Moreover, the recombinant antibody protein on the obtained composite lipid nanoparticle still has a good ability to specifically recognize and bind a corresponding protein.

## Description

### TECHNICAL FIELD

The present disclosure belongs to the technical field of lipid nanoparticle biological application, and particularly to a composite lipid nanoparticle and a preparation method thereof, a ribose nucleic acid (RNA) vaccine, a drug and use.

### BACKGROUND

A lipid nanoparticle (LNP) is a novel nanotechnology product, which is generally composed of four components, i.e., a phospholipid, cholesterol, a polymer modified lipid and an ionic lipid, the above components jointly form a dual-layer structure that is stable and similar to a cell membrane. LNP can encapsulate and protect mRNA, and meanwhile the linkage of a ligand protein of a target site to LNP helps mRNA and a drug precisely reach and act on a target cell or tissue. Moreover, the material used by LNP can be safely decomposed in vivo when pH changes, and therefore LNP is widely used in the field of nucleic acid and drug delivery.

At present, the linkage between LNP and a protein is achieved by using a covalent linkage method. According to a reaction mechanism, the covalent linkage method specifically includes a maleimide-thiol reaction method, a click chemistry method, and an amino and carboxyl cross-linking method. The maleimide-thiol reaction method is that a maleimide group is introduced on the surface of LNP, and the maleimide group and the thiol group (-SH) that naturally exists or is introduced by engineering in the protein form a stable covalent bond, so as to achieve the stable linkage of the protein. The click chemistry method is that alkynyl and azide groups are introduced on LNP and a protein respectively, and a covalent bond is rapidly formed through azide-alkyne click chemistry. The amino and carboxyl cross-linking method is that the carboxyl on the surface of LNP or the amino in the protein is activated, and a coupling reaction is carried out by utilizing EDC or NHS so as to form an amido bond between LNP and the protein, thereby achieving the linkage.

However, the above-described covalent linkage has issues associate with poor stability, non-specific linkage, low linkage efficiency, residual chemical reagents, high operation condition requirements and the like, and therefore has great restriction.

### SUMMARY

The first objective of the present disclosure is to provide a composite lipid nanoparticle in order to solve the problems in the existing methods for achieving the linkage of LNP and a protein, such as poor stability, non-specific linkage, low linkage efficiency, residual chemical reagents and high operation condition requirements. Through strong interaction between a recombinant antibody protein with a streptavidin tag and a biotinylated polyethylene glycol lipid in the composite lipid nanoparticle, the safe, convenient, rapid, efficient and stable linkage between LNP and the recombinant antibody protein is achieved and the recombinant antibody protein can be adaptively replaced according to a target cell and/or tissue, with high flexibility and applicability. Moreover, the recombinant antibody protein on the obtained composite lipid nanoparticle still has a good ability to specifically recognize and bind a corresponding protein.

The second objective of the present disclosure is to provide a preparation method of the above described composite lipid nanoparticle.

The third objective of the present disclosure is to provide an RNA vaccine.

The fourth objective of the present disclosure is to provide a drug.

The fifth objective of the present disclosure is to provide use of the above described composite lipid nanoparticle in the field of nucleic acid and drug delivery.

The composite lipid nanoparticle provided by the present disclosure comprises: a lipid composition and a recombinant antibody protein with a streptavidin tag; the lipid composition comprises an ionizable lipid, an auxiliary lipid, cholesterol, a polyethylene glycol lipid and a biotinylated polyethylene glycol lipid, and a mass ratio of the ionizable lipid to the auxiliary lipid to the cholesterol to the polyethylene glycol lipid to the biotinylated polyethylene glycol lipid is (40-90):(0.1-20):(20-50):(0.5-10):(0.5-10).

In some specific embodiments, the ionizable lipid is selected from one or more of trimethyl-2,3-dioleoyloxypropyl ammonium bromide, trimethyl-2,3-diolephenoxypropyl ammonium chloride, 3β-[N-(N',N'-dimethylaminoethyl) aminoformyl] and 8-[(2-hydroxyethyl)[6-oxo-6-(undecyloxy)hexyl]amino]-octanoic acid, 1-octylnonyl ester.

In some specific embodiments, the auxiliary lipid is selected from one or more of dioleoyl phosphatidyl ethanolamine, stearoyl phosphatidylcholine, distearoyl phosphatidylcholine and sterol.

In some specific embodiments, the polyethylene glycol lipid is selected from one or more of 1,2-dimyristoyl-rac-glycerin-3-methoxy polyethylene glycol, phosphatidylethanolamine distearate-polyethylene glycol and dioleoyl phosphatidylethanolamine-polyethylene glycol.

In some specific embodiments, the biotinylated polyethylene glycol lipid is selected from distearoyl phosphatidylethanolamine-polyethylene glycol-biotin and/or 1,2-dimyristoyl-rac-glycerin-3-methoxy polyethylene glycol-biotin.

In some specific embodiments, the biotinylated polyethylene glycol lipid is selected from one or more of biotinylated polyethylene glycol modified phospholipid, biotinylated polyethylene glycol modified cholesterol and biotinylated polyethylene glycol modified fatty acid.

In some specific embodiments, the composite lipid nanoparticle coats a nucleic acid molecule and/or a drug molecule.

In some specific embodiments, the composite lipid nanoparticle coats the nucleic acid molecule, and a molar ratio of nitrogen in an ionizable lipid to a phosphoric acid in an nucleic acid molecule is (4-6): 1.

In some specific embodiments, the preparation of the recombinant antibody protein with the streptavidin tag comprises: constructing with a streptavidin encoding gene and an antibody protein encoding gene by using a seamless cloning technology to obtain a seamlessly cloned fragment; and expressing the seamlessly cloned fragment to obtain the recombinant antibody protein with the streptavidin tag.

In some specific embodiments, the seamless cloning technology comprises: taking an expression vector for an enzyme-digested treatment to obtain an enzyme-digested product; performing polymerase chain reaction (PCR) amplification on the enzyme-digested product, the streptavidin encoding gene and the antibody protein encoding gene to obtain a PCR amplification product; and transforming and screening the PCR amplification product to obtain the seamlessly cloned fragment.

In some specific embodiments, the expression vector is selected from a pCAGGS vector and/or a pGL3 vector.

In some specific embodiments, the expression vector is the pCAGGS vector, and a primer system for the seamless cloning comprises: SA-F1 with a nucleotide sequence as shown in SEQ ID NO:1 and SA-R1 with a nucleotide sequence as shown in SEQ ID NO:2; and/or, Ab-F1 with a nucleotide sequence as shown in SEQ ID NO:3 and Ab-R1 with a nucleotide sequence as shown in SEQ ID NO:4.

A preparation method of the composite lipid nanoparticle provided by the present disclosure comprises: mixing the lipid composition with ethanol to obtain an organic phase solution; and mixing the recombinant antibody protein with the streptavidin tag, an optional nucleic acid molecule and an optional drug with water to obtain a water phase solution; and mixing the organic phase solution with the water phase solution to obtain the composite lipid nanoparticle.

In some specific embodiments, the concentration of the lipid composition in the organic phase solution is 50-75wt%.

In some specific embodiments, the concentration of the recombinant antibody protein in the water phase solution is 20-30wt%, and a mixing volume ratio of the organic phase solution to the water phase solution is 1: (1-5).

The RNA vaccine provided by the present disclosure comprises the above described composite lipid nanoparticle.

The drug provided by the present disclosure comprises the above described composite lipid nanoparticle.

The present disclosure provides use of the above described composite lipid nanoparticle in nucleic acid and/or drug delivery.

The beneficial effects:

The present disclosure provides a composite lipid nanoparticle comprising a lipid composition containing a biotinylated polyethylene glycol lipid and a recombinant antibody protein with a streptavidin tag. Firstly, by utilizing strong specific interaction between biotin and streptavidin, the linkage between the lipid nanoparticle and the protein can be achieved through simple operations such as mixing; secondly, by using a binding strategy of introducing biotin into the lipid nanoparticle and introducing streptavidin on the antibody protein, the recombinant antibody protein binds to multiple biotinylated polyethylene glycol lipids, thereby achieving a more stable linkage between the lipid nanoparticle and the antibody protein; moreover, introducing the streptavidin tag whose chemical nature is a protein into the antibody protein does not disturb the ability of the antibody protein to specifically recognize the corresponding protein, and such the feature ensures that the composite lipid nanoparticle can precisely guide the target cell and/or tissue, thereby achieving the precise delivery of the drug, with better tissue delivery efficiency.

### BRIEF DESCRIPTION OF THE DRAWINGS

FIG. 1 is a scanning electron microscopy (SEM) image (a scale is 200 nm) of a composite lipid nanoparticle provided in example 1 according to the present disclosure.
FIG. 2 is an experimental result graph showing a composite lipid nanoparticle transfects macrophages provided in test example according to the present disclosure.
FIG. 3 is an experimental result graph showing a composite lipid nanoparticle transfects mice provided in test example according to the present disclosure.

### DETAILED DESCRIPTION OF THE EMBODIMENTS

The composite lipid nanoparticle provided in the present disclosure specifically comprises a lipid composition and a recombinant antibody protein with a streptavidin tag. Wherein the lipid composition comprises an ionizable lipid, an auxiliary lipid, cholesterol, a polyethylene glycol lipid and a biotinylated polyethylene glycol lipid; and a mass ratio of the ionizable lipid to the auxiliary lipid to the cholesterol to the polyethylene glycol lipid to the biotinylated polyethylene glycol lipid is (40-90):(0.1-20):(20-50):(0.5-10):(0.5-10), such as 40:0.1:20:0.5:0.5, 45:1:30:3:4, 75:5:37:6:10, 80:13:45:8:7, 90:20:50:10:5, or any value of them. The recombinant antibody protein with the streptavidin tag is prepared by recombining and expressing a streptavidin encoding gene and an antibody protein encoding gene utilizing a biotechnology method, so as to achieve the splicing of a streptavidin protein and an antibody protein; at this moment, compared with an existing fact that chemical group modification is performed on the antibody protein to endow the antibody protein with reactivity with a lipid nanoparticle, the antibody protein provided by the present disclosure can better maintain the ability of the antibody protein to specifically recognize the corresponding protein.

In the present disclosure, the ionizable lipid is a type of lipids having one or more hydrophilic groups and one or more hydrophobic groups. Ionization performance inside and outside the cell endows the ionizable lipid with an ability to freely pass through the cell membrane. The ionizable lipid is a type of substance commonly used in the existing lipid nanoparticles, and is not specifically limited. In some specific embodiments, the specific examples of the ionizable lipid include but are not limited to one or more of trimethyl-2,3-dioleoyloxypropyl ammonium bromide, trimethyl-2,3-diolealkoxypropyl ammonium chloride, 3β-[N-(N',N'-dimethylaminoethyl) aminoformyl] and 8-[(2-hydroxyethyl) [6-oxo-6-(undecyloxy) hexyl] amino] octanoic acid 1-octylnonyl ester.

In the present disclosure, the auxiliary lipid is a type of neutral lipids taking the effects of improving stability and improving internal circulation in lipid nanoparticles, which is a type of substance commonly used in the existing lipid nanoparticles and are not specifically limited. In some specific embodiments, the specific examples of the auxiliary lipid include but are not limited to one or more of dioleyl phosphatidyl ethanolamine, stearoyl phosphatidylcholine, di-stearoyl phosphatidylcholine and sterol.

In the present disclosure, the polyethylene glycol lipid is a long chain structure with amphiphilicity, which is composed of a hydrophilic polyethylene glycol chain and a hydrophobic alkyl and/or dialkyl chain. The addition of the polyethylene glycol lipid can not only improve the particle size and particle stability of the composite lipid nanoparticle, but also affect the nucleic acid encapsulation efficiency, half-life, transfection efficiency, immune response and the like of the composite lipid nanoparticle. The polyethylene glycol lipid is a type of substance commonly used in the existing lipid nanoparticles and is not limited. In some specific embodiments, the specific examples of the polyethylene glycol lipid include but are not limited to one or more of 1,2-dimyristoyl-rac-glycerin-3-methoxy polyethylene glycol, phosphatidylethanolamine distearate-polyethylene glycol and dioleoyl phosphatidylethanolamine-polyethylene glycol.

In the present disclosure, the biotinylated polyethylene glycol lipid is a type of molecule obtained by linking biotin to a polyethylene glycol lipid structure, which achieves the rapid linkage with the antibody protein by utilizing strong binding between biotin and streptavidin, is prepared by modifying the polyethylene glycol lipid with biotin, and the modification of the polyethylene glycol lipid with biotin is a method commonly used in the prior art, and is not specifically limited. In some specific embodiments, the specific examples of the biotinylated polyethylene glycol lipid include but are not limited to distearoyl phosphatidylethanolamine-polyethylene glycol-biotin and/or 1,2-di-myristoyl-rac-glycerin-3-methoxy polyethylene glycol-biotin.

In the present disclosure, based on the weight of the lipid composition, the content of the ionizable lipid is preferably 40-60wt%, such as 40wt%, 43wt%, 48wt%, 50wt%, 51.5wt%, 53wt%, 55wt%, 58wt%, 60wt%, or any value of them; the content of the auxiliary lipid is preferably 5-20wt%, such as 5wt%, 5.8wt%, 6wt%, 6.5wt%, 7wt%, 9wt%, 10wt%, 12.5wt%, 14wt%, 18wt%, 20wt%, or any value of them; the content of the cholesterol is preferably 30-50wt%, such as 30wt%, 30.5wt%, 32wt%, 35wt%, 38wt%, 40wt%, 43wt%, 45wt%, 50wt%, or any value of them; the content of the polyethylene glycol lipid is preferably 0.1-20wt%, such as 0.1wt%, 0.5wt%, 0.8wt%, 1wt%, 1.3wt%, 1.8wt%, 2wt%, 5wt%, 9wt%, 15wt%, 18wt%, 20wt%, or any value of them; the content of the biotinylated polyethylene glycol lipid is preferably 0.1-20wt%, such as 0.1wt%, 1wt%, 3wt%, 7wt%, 10wt%, 13wt%, 18wt%, 20wt%, or any value of them.

In the present disclosure, the composite lipid nanoparticle preferably coats a nucleic acid molecule and/or a drug molecule according to expected application scenarios. In some specific embodiments, the composite lipid nanoparticle is used for delivery of nucleic acid molecules such as DNA, mRNA, siRNA and circRNA, and a molar ratio of nitrogen in an ionizable lipid to a phosphoric acid in an nucleic acid molecule is (4-6):1. Such as, 4:1, 4.05:1, 4.3:1, 4.8:1, 5:1, 5.5:1, 6:1, or any value of them.

In the present disclosure, the preparation of the recombinant antibody protein with the streptavidin tag preferably comprises: constructing with a streptavidin encoding gene and an antibody protein encoding gene by using a seamless cloning technology to obtain a seamlessly cloned fragment; and expressing the seamlessly cloned fragment to obtain the recombinant antibody protein with the streptavidin tag.

In the present disclosure, the seamless cloning technology is a biological technology applicable for achieving the splicing of a plasmid vector linearized by any method and one or more DNA fragment and constructing multi-site mutation, which has the advantages of rapid, convenient, efficient and multi-fragment assembling, directed cloning and the like.

In the present disclosure, the seamless cloning technology preferably comprises: taking an expression vector for an enzyme-digested treatment to obtain an enzyme-digested product; performing polymerase chain reaction (PCR) amplification on the enzyme-digested product, the streptavidin encoding gene and the antibody protein encoding gene to obtain a PCR amplification product; and transforming and screening the PCR amplification product to obtain the seamlessly cloned fragment.

In some specific embodiments, an enzyme-digested system for the enzyme-digested reaction includes but is not limited to a restriction endonuclease and/or a T5 exonuclease. The conditions for the enzyme-digested reaction include a temperature, preferably 30-35°C, such as 30°C, 30.5°C, 31°C, 32°C, 33°C, 35°C, or any value of them; time, preferably 30-60 min, such as 30 min, 35 min, 38 min, 45 min, 50 min, 55 min, 60 min, or any value of them.

In some specific embodiments, the expression vector is a type of substance that is commonly used in the seamless cloning technology and is not specifically limited. The specific examples of the expression vector include but are not limited to a pCAGGS vector and/or a pGL3 vector. In some more preferred embodiments, the expression vector is preferably the pCAGGS vector, a primer system for the seamless cloning preferably including SA-F1 with a nucleotide sequence as shown in SEQ ID NO:1 and SA-R1 with a nucleotide sequence as shown in SEQ ID NO:2; and/or, Ab-F1 with a nucleotide sequence as shown in SEQ ID NO:3 and Ab-R1 with a nucleotide sequence as shown in SEQ ID NO:4. At this moment, the recombinant antibody protein with the streptavidin tag, which is expressed by the seamlessly cloned fragment obtained after PCR amplification, transformation and screening using the primer system, has a better ability to specifically bind to a relevant protein.

A preparation method of the composite lipid nanoparticle provided by the present disclosure specifically comprises: mixing the lipid composition with ethanol to obtain an organic phase solution; and mixing the recombinant antibody protein with the streptavidin tag, an optional nucleic acid molecule and an optional drug with water to obtain a water phase solution; and mixing the organic phase solution with the water phase solution to obtain the composite lipid nanoparticle.

In the present disclosure, an addition mass ratio of the lipid composition to the ethanol is preferably the concentration of the lipid composition in the organic phase solution, which is 50-75wt%, such as 50wt%, 55wt%, 58wt%, 60wt%, 65wt%, 70wt%, 75wt%, or any value of them. At this moment, the concentration of the lipid composition in the organic phase solution is appropriate, which is beneficial for the formation of the composite lipid nanoparticle with good morphology and high particle size uniformity.

In the present disclosure, the composite lipid nanoparticle is gradually separated out and solidified in the process of mixing the organic phase solution with the water phase solution while achieving the insertion of the recombinant antibody protein with the streptavidin tag and entrapment of the nucleic acid molecule and/or drug; the method for mixing the organic phase solution with the water phase solution is a method commonly used in the preparation of the existing composite lipid nanoparticle and is not specifically limited, and its specific examples include but are not limited to one or more of a microfluidic mixing technology, a thin film hydration method, an extrusion method, an injection method and a homogenization method. In some specific embodiments, the concentration of the recombinant antibody protein with the streptavidin tag in the water phase solution is 20-30wt%, such as 20wt%, 21wt%, 23wt%, 25wt%, 27.5wt%, 28wt%, 30wt%, or any value of them, a mixing volume ratio of the organic phase solution to the water phase solution is preferably 1:(1-5), such as 1:1, 1:1.5, 1:2, 1:3, 1:4, 1:5, or any value of them. At this moment, the ratio of the recombinant protein to the lipid composition and the ratio of the organic phase solution to the water phase solution are appropriate, which leads to the formation of ideal recombinant antibody protein distribution and a three-dimensional structure on the composite lipid nanoparticle, so as to achieve a better drug delivery effect.

The RNA vaccine provided by the present disclosure specifically comprises the above described composite nanoparticle. The entrapment of RNA in the composite lipid nanoparticle results in safe and stable delivery of RNA to a target cell and/or tissue, thereby achieving good immune effect.

The drug provided by the present disclosure specifically comprises the above described composite lipid nanoparticle. The entrapment of the drug molecule in the composite lipid nanoparticle results in safe and stable delivery of the drug molecule to a target cell and/or tissue, thereby achieving good immune effect.

The present disclosure also provides use of the above described composite lipid nanoparticle in the field of nucleic acid and/or drug delivery.

Next, the embodiments of the present disclosure will be described in detail. The examples of the embodiments are intended to explain the present disclosure, but cannot be understood as limiting the present disclosure. Specific technologies or conditions that are not noted in the embodiments are carried out according to the technologies or conditions described in literatures in the prior art. The reagents or instruments used without specifying the manufacturer are conventional products that can be obtained through commercial purchase.

The reagents and their sources involved in the following examples and comparative examples are as follows:
The LB solid culture medium containing ampicillin sodium (Sangon Biotech, Article number: B530112);
The ionizable lipid (AVT, Article number:O02010);
The auxiliary lipid (AVT, Article number: DSPC);
Cholesterol (Sigma, Article number: C8667);
Polyethylene glycol lipid (AVT, Article number: 002005);
The biotinylated polyethylene glycol lipid (Xi'an Ruixi Biotechnology, Article number: R-1304-2k);
DEME culture medium (Gbico, Article number: C11995500BT);
Streptavidin (Yeasen, Article number: 35100ES03).

Nucleic acids involved in examples and comparative examples below are as shown in Table 1.

**Table 1 Nucleotide sequence listing**

| Name | SEQ ID NO: | Nucleotide sequence |
|---|---|---|
| Primer SA-F1 | 1 | GCTGAGGCCGGCATCACCG |
| Primer SA-R1 | 2 | AAAGATCTGCTAGCTCGAGTCAGTGGTGGTGGTGG TGGTGATGATG |
| Primer Ab-F1 | 3 | 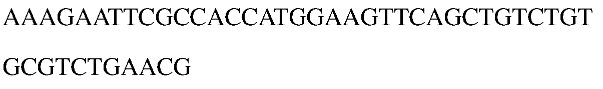 |
| Primer Ab-R1 | 4 | TTTGATTTCCAGTTTAGTACCACCACCGAAGG |
| Streptavidin encoding gene | 5 | GCTGAGGCCGGCATCACCGGCACATGGTACAACCA GAGAGGCTCTACCTTTACCGTGACCGCCGGAGCTG ACGGTAACCTTACAGGCCAGTATGAGAACCGCGCA CAGGGCTGGGGCTGTCAGAATAGTCCATACACACT CACAGGTAGGTACAACGGCACCAAGTTGGAATGG AGAGTGGAATGGAACAACTCTACCGAGAATTGCCA CTCCAGAACCGAGTGGAGAGGCCAGTACCAGGGA GGTGCTGAGGCCAGAATCAATACACAGTGGAACCT |
| | | GACATACGAGGGTGGCAGTGGTCCAGCCACCACA CAGGGTCAAGACACCTTCACCAAGGTCAAGCCTTC TGCTGCATCTCATCATCACCACCACCACCACCAC |
| Antibody protein encoding gene | 6 | GAAGTTCAGCTGTCTGTGCGTCTGAACGGTGAAGC GTGGGGTTTCTCCGAAGACAGCTGCAAAGCGTCTG GTTACACCTTCACCGACTACAACATGGACTGGGTT AAACAGAGCCACGAAAAGTCTCTGGAATGGATCG GTTACATCAATCCGTATAGCGGTGACACCATCTACA ACCATAAATTCAAAGATAAAGCGACTCTGACCGTT GATAAATCTTCCAACATCGCGTACATGGAACTGCGC AGCTTGACCTCTGAAGACACTGCTGTTTACTACTG CGCGCGTGGTGGTTACGATTACGGTGATCACTGGG GCCAGGGTACTACTCTGACTGTGTCCAGCGGTTCT GCGGGTGGTGGTGGTTCTGGTGGTGGCGGTAGCGG TGGCGGTGGTTCCGACATCAAGATGACTCAGAGTC CATCTTCTATGTACGCAAGCCTGGGTGAACGTGTTA CCATCACCTGCAAAGCATCTCAAGACATCAACTCT TACCTGTCTTGGTTCCAGCAGAAACCGGGCAAGAG TCCGAAGACTCTGATCTACCGTGCCAACCGTCTGG TAGGCGGTGTACCGTCTCGTTTCTCCGGTTCTGGTT CCGGTCAAGACTACAGCCTGACCATCAGCAGCCTG GAATATGAAGACATGGGCATCTACTACTGCCTGCAA GACGACGAATTTCCGCGTACCTTCGGTGGTGGTAC TAAACTGGAAATCAAA |

### Example 1

This example is used for illustrating the preparation of a composite lipid nanoparticle, which specifically comprises:
1. The construction of a recombinant antibody protein with a streptavidin tag: (1) a streptomycin encoding gene with a nucleotide sequence as shown in SEQ ID NO: 5 and an antibody protein encoding gene with a nucleotide sequence as shown in SEQ ID NO: 6 were obtained by gene synthesis, and a PCAGGS vector was subjected to an enzyme-digested treatment using restriction endonuclease for 30 min at 37°C to obtain an enzyme-digested product;
   (2) PCR amplification was performed on the streptavidin encoding gene and the antibody protein encoding gene by using SA-F1 with a nucleotide sequence as shown in SEQ ID NO: 1, SA-R1 with a nucleotide sequence as shown in SEQ ID NO: 2, Ab-F1 with a nucleotide sequence as shown in SEQ ID NO: 3 and Ab-R1 with a nucleotide sequence as shown in SEQ ID NO: 4 to obtain PCR amplification product;
      A PCR reaction system included: 1 µL of streptavidin encoding gene or 1 µL of antibody protein encoding gene, 1 µL of SA-F1, 1 µL of SA-R1, or 1 µL of Ab-F1, 1 µL of Ab-R1, 10 µL of 5 × fast pfu buffer, 4 µL of dNTPs (2.5 mM), 1.5 µL of Fast pfu and ddH₂O were added until a total volume was 50 µL; reaction conditions included: pre-denaturation for 60 s at 94°C, denaturation for 30 s at 94°C, annealing for 30 s at 58°C, extending for 5 min at 72°C, and 30 cycles.
   (3) Escherichia coli DH5α competent cells (with a cell density of 70%) were transfected with 5 µL of PCR amplification product for 24 h, then the transfected Escherichia coli DH5α competent cells were coated onto an LB solid culture medium containing ampicillin sodium, and then a well-grown single colony was picked and sequenced for identification and screening, so as to obtain a seamlessly cloned fragment;
   (4) 293f cells (with a cell density of 70%) were transfected with the seamlessly cloned fragment for 96 h, then a culture solution was collected and subjected to gel electrophoresis purification to obtain the recombinant antibody protein with the streptavidin tag.
2. The preparation of a composite lipid nanoparticle using an ethanol injection method: (1) 275 mass parts of ionizable lipid, 70 mass parts of auxiliary lipid, 120 mass parts of cholesterol, 7.5 mass parts of polyethylene glycol lipid, 9 mass parts of biotinylated polyethylene glycol lipid and 100 mass parts of ethanol were sufficiently mixed to obtain an organic phase solution;
   (2) 100 mass parts of recombinant antibody protein with the streptavidin tag, 20 mass parts of luciferase-CY5 mRNA and 300 mass parts of water were sufficiently mixed to obtain a water phase solution;
   (3) the water phase solution was rapidly added into the organic phase solution in a volume ratio of 3:1 (water phase solution to the organic phase solution), the above two solutions were evenly mixed and then incubated for 30 min to obtain a suspension containing a composite lipid nanoparticle, and the suspension was ultra-filtrated and centrifuged to obtain the composite lipid nanoparticle.

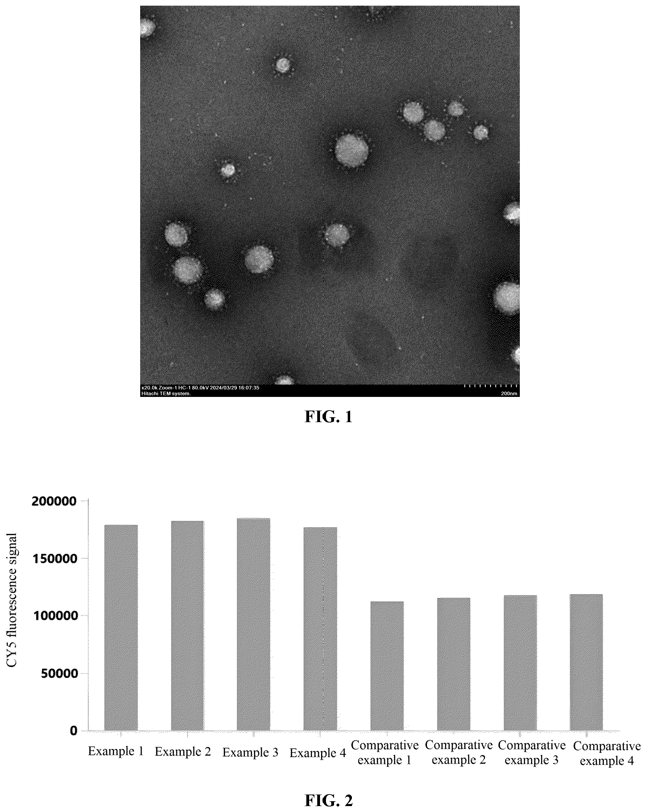

FIG. 1 is an SEM image of a composite lipid nanoparticle prepared in the present disclosure. It can be seen from FIG. 1 that the composite lipid nanoparticle has a regular spherical structure, and a certain amount of recombinant antibody proteins with the streptavidin tag are successfully linked to the surface of the composite lipid nanoparticle.

### Example 2

A composite lipid nanoparticle was prepared according to the method provided in example 1 except that in (1) of step 2 in this example, 40.75 mass parts of ionizable lipid and 10 mass parts of biotinylated polyethylene glycol lipid were added, and the other conditions were the same, so as to obtain the composite lipid nanoparticle.

### Example 3

A composite lipid nanoparticle was prepared according to the method provided in example 1 except that in (1) of step 2 in this example, 65 mass parts of ionizable lipid, 8 mass parts of auxiliary lipid and 25.5 mass parts of cholesterol were added, and other conditions were the same, so as to obtain the composite lipid nanoparticle.

### Example 4

A composite lipid nanoparticle was prepared according to the method provided in example 1 except that in (3) of step 2 in this example, a volume ratio of a water phase solution to an organic phase solution was 4:1, and other conditions were the same, so as to obtain the composite lipid nanoparticle.

### Comparative example 1

In this comparative example, a composite lipid nanoparticle was prepared according to the method provided in example 1 except that in (1) of step 2 in this example, the recombinant antibody protein with the streptavidin tag was replaced with an equal mass part of biotinylated antibody protein, 15 mass parts of streptavidin were added, and other conditions were the same, so as to obtain the composite lipid nanoparticle.

Wherein, the preparation of the biotinylated antibody protein included: an antibody protein encoding gene with a nucleotide sequence as shown in SEQ ID NO:6 was obtained by gene synthesis, a PCAGG3 vector was digested with restriction endonuclease at 37°C for 30 min to obtain an enzyme-digested product, and according to the method for construction of a recombinant antibody protein with a streptavidin tag provided in example 1, the antibody protein was expressed;
subsequently, the antibody protein was transferred to 1×modification buffer (containing 100 mM of phosphoric acid and 150 mM of sodium chloride, pH 7.2-7.4) to formulate an antibody solution with a protein concentration of 5 mg/L; 0.8 L of biotin solution (a dimethylformamide solution containing 20 g/L biotin) was added into 1 L of antibody solution, and then the above two solutions were stirred and incubated for 2 h at a room temperature, centrifuged and filtrated, so as to obtain a solution containing biotinylated antibody proteins.

### Comparative example 2

In this comparative example, a composite lipid nanoparticle was prepared according to the method provided in example 1 except that in (1) of step 2, the biotinylated polyethylene glycol lipid was replaced with an equal mass part of polyethylene glycol lipid, and other conditions were the same, so as to obtain the composite lipid nanoparticle.

### Comparative example 3

In this comparative example, a composite lipid nanoparticle was prepared according to the method provided in example 1 except that in (2) of step 2, a recombinant antibody protein with a streptavidin tag was replaced with an equal mass part of antibody protein, and other conditions were the same, so as to obtain the composite lipid nanoparticle.

Wherein, the preparation of the antibody protein refers to comparative example 1.

### Comparative example 4

In this comparative example, a composite lipid nanoparticle was prepared according to the method provided in example 1 except that in (2) of step 2, a recombinant antibody protein with a streptavidin tag was replaced with an equal mass part of water, and other conditions were the same, so as to obtain the composite lipid nanoparticle.

### Test example

This test example is used for illustrating delivery efficiency and immunogenicity of composite lipid nanoparticles provided in examples 1-4 and comparative examples 1-4, and specifically comprises:
1. Transfection of macrophages with composite lipid nanoparticles: (1) macrophages were added into a DEME culture medium and cultured in a CO₂ incubator under the conditions of 5% CO₂, saturated humidity and 37°C, until the density of cells was about 70%;
(2) 0.2 µg of composite lipid nanoparticles were added into a culture medium and incubated for 4 h in a CO₂ incubator under the conditions of 5% CO₂, saturated humidity and 37°C, and a CY5 fluorescence signal was detected. The test results are as shown in Table 2 and FIG. 2.

**Table 2 Ability of composite lipid nanoparticle to transfect macrophages**

| Group | CY5 fluorescence signal |
|---|---|
| Example 1 | 178500 |
| Example 2 | 182000 |
| Example 3 | 184000 |
| Example 4 | 176000 |
| Comparative example 1 | 112000 |
| Comparative example 2 | 115000 |
| Comparative example 3 | 117000 |
| Comparative example 4 | 118000 |

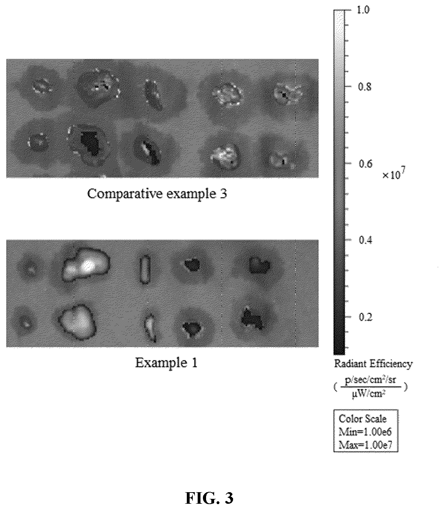

It can be seen from test results of Table 2 and FIG. 2 that compared with comparative examples 1-4, the CY5 fluorescence signal presented by the macrophages transfected with the composite lipid nanoparticles provided in examples 1-4 is at least 1.4 times higher than that in comparative examples 1-4, indicating that the composite lipid nanoparticles provided in examples 1-4 can better recognize and bind macrophages, and achieve an ideal transfection effect.
2. Transfection of mice with composite lipid nanoparticles: BALB/c nude mice (male, 6-8 weeks old, SPF grade feeding conditions) were used as experimental animals, the right abdominal cavities of the nude mice were inserted with a needle by about 5 mm, 5 µg of composite lipid nanoparticles from example 1 and comparative example 3 were injected intraperitoneally, the needle was rotated and withdrawn after injection, and a pinhole was pressed with a cotton swab until no liquid flowed out so as to complete intraperitoneal injection, the mice were dissected on 1st day after injection, and fluorescence imaging was performed on the hearts, livers, spleens, lungs, and kidneys (organs) of the mice. The results are shown in FIG. 3.

It can be seen from FIG. 3 that under the same mouse cultivation treatment conditions, compared with comparative example 3, the mice are transfected with the composite lipid nanoparticles provided in example 1, the liver, spleen, lung and kidney (organs) present stronger fluorescence signals, that is to say, the luciferase-CY5 mRNA coated with the composite lipid nanoparticles has better delivery efficiency on the mice.

Although the above descriptions have shown and described the embodiments of the present disclosure, it can be understood that the above embodiments are exemplary, and cannot be understood as limiting the present disclosure. Changes, modifications, replacements and deformations can be made to the above described embodiments by the persons of ordinary skill in the art within the scope of the present disclosure without departing from the principle and spirit of the present disclosure.

## Claims

1. A composite lipid nanoparticle, wherein the composite lipid nanoparticle comprises a lipid composition and a recombinant antibody protein with a streptavidin tag;
the lipid composition comprises an ionizable lipid, an auxiliary lipid, cholesterol, a polyethylene glycol lipid and a biotinylated polyethylene glycol lipid, and a mass ratio of the ionizable lipid to the auxiliary lipid to the cholesterol to the polyethylene glycol lipid to the biotinylated polyethylene glycol lipid is (40-90):(0.1-20):(20-50):(0.5-10):(0.5-10).

2. The composite lipid nanoparticle according to claim 1, wherein the ionizable lipid is selected from one or more of trimethyl-2,3-dioleoyloxypropyl ammonium bromide, trimethyl-2,3-diolephenoxypropyl ammonium chloride, 3β-[N-(N',N'-dimethylaminoethyl) aminoformyl] and 8-[(2-hydroxyethyl)[6-oxo-6-(undecyloxy) hexyl] amino] octanoic acid 1-octylnonyl ester.

3. The composite lipid nanoparticle according to claim 1, wherein the auxiliary lipid is selected from one or more of dioleoyl phosphatidyl ethanolamine, stearoyl phosphatidylcholine, di-stearoyl phosphatidylcholine and sterol.

4. The composite lipid nanoparticle according to claim 1, wherein the polyethylene glycol lipid is selected from one or more of 1,2-dimyristoyl-rac-glycerin-3-methoxy polyethylene glycol, phosphatidylethanolamine distearate-polyethylene glycol and dioleoyl phosphatidylethanolamine-polyethylene glycol.

5. The composite lipid nanoparticle according to claim 1, wherein the biotinylated polyethylene glycol lipid is selected from distearoyl phosphatidylethanolamine-polyethylene glycol-biotin and/or 1,2-dimyristoyl-rac-glycerin-3-methoxy polyethylene glycol-biotin.

6. The composite lipid nanoparticle according to claim 1, wherein based on the weight of the lipid composition, the content of the ionizable lipid is 40-60wt%, the content of the auxiliary lipid is 5-20wt%, the content of the cholesterol is 30-50wt%, the content of the polyethlene glycol lipid is 0.1-20wt%, and the content of the biotinylated polyethylene glycol lipid is 0.1-20wt%.

7. The composite lipid nanoparticle according to claim 1, wherein the composite nanoparticle coats a nucleic acid molecule and/or a drug molecule.

8. The composite lipid nanoparticle according to claim 7, wherein the composite lipid nanoparticle coats the nucleic acid molecule, and a molar ratio of nitrogen in an ionizable lipid to a phosphoric acid in an nucleic acid molecule is (4-6): 1.

9. The composite lipid nanoparticle according to claim 1, wherein the preparation of the recombinant antibody protein with the streptavidin tag comprises: constructing with a streptavidin encoding gene and an antibody protein encoding gene by using a seamless cloning technology to obtain a seamlessly cloned fragment; and expressing the seamlessly cloned fragment to obtain the recombinant antibody protein with the streptavidin tag.

10. The composite lipid nanoparticle according to claim 9, wherein the seamless cloning technology comprises: taking an expression vector for an enzyme-digested treatment to obtain an enzyme-digested product; performing polymerase chain reaction (PCR) amplification on the enzyme-digested product, the streptavidin encoding gene and the antibody protein encoding gene to obtain a PCR amplification product; and transforming and screening the PCR amplification product to obtain the seamlessly cloned fragment.

11. The composite lipid nanoparticle according to claim 10, wherein the expression vector is selected from a pCAGGS vector and/or a pGL3 vector.

12. The composite lipid nanoparticle according to claim 10, wherein the expression vector is the pCAGGS vector, and a primer system for the seamless cloning comprises: SA-F1 with a nucleotide sequence as shown in SEQ ID NO:1 and SA-R1 with a nucleotide sequence as shown in SEQ ID NO:2; and/or, Ab-F1 with a nucleotide sequence as shown in SEQ ID NO: 3 and Ab-R1 with a nucleotide sequence as shown in SEQ ID NO:4.

13. A preparation method of the composite lipid nanoparticle according to claims 1, wherein the method comprises: mixing the lipid composition with ethanol to obtain an organic phase solution; and mixing the recombinant antibody protein with the streptavidin tag, an optional nucleic acid molecule and an optional drug with water to obtain a water phase solution; and mixing the organic phase solution with the water phase solution to obtain the composite lipid nanoparticle.

14. The preparation method of the composite lipid nanoparticle according to claim 13, wherein the concentration of the lipid composition in the organic phase solution is 50-75wt%.

15. The preparation method of the composite lipid nanoparticle according to claim 13, wherein the concentration of the recombinant antibody protein with the streptavidin tag in the water phase solution is 20-30wt%, and a mixing volume ratio of the organic phase solution to the water phase solution is 1: (1-5).

16. A ribose nucleic acid (RNA) vaccine, comprising the composite lipid nanoparticle according to claims 1.

17. A drug, comprising the composite lipid nanoparticle according to claims 1.

18. Use of the composite lipid nanoparticle according to claim 1 in the field of nucleic acid and/or drug delivery.
